(19) Europäisches Patentamt
European Patent Office
Office européen des brevets

(11) EP 1 997 914 A1

(12) EUROPEAN PATENT APPLICATION

(43) Date of publication:
**03.12.2008 Bulletin 2008/49**

(51) Int Cl.:
***C12Q 1/70*** *(2006.01)*

(21) Application number: **07109454.4**

(22) Date of filing: **01.06.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Università Degli Studi Di Milano - Bicocca**
**20126 Milano (IT)**

(72) Inventors:
- **Cocuzza, Clementina Elvezia Anna**
**20146 Milano (IT)**
- **Broccolo, Francesco**
**20040 Busnago (MI) (IT)**

(74) Representative: **Minoja, Fabrizio**
**Bianchetti Bracco Minoja S.r.l.**
**Via Plinio, 63**
**20129 Milano (IT)**

(54) **Identification and quantification of oncogenic HPV nucleic acids by means of real-time PCR assays**

(57) Method for the identification and quantification of oncogenic HPV nucleic acids comprising:
a) first line screening by means of a SYBR Green I Real-time PCR assay to determine the total viral DNA load and to identify the presence of one or more of 13 high risk HPV genotypes in clinical samples;
b) second line assays to be applied to samples positives for a):
- 4 independent TaqMan Real-time PCR assays to determine the presence and the viral load of the most common oncogenic HPV types: 16, 18 and/or 45, 31, 33 and/or 58;
- 6 independent SYBR Green I RT Real-time PCR assays to determine the presence in the sample of the oncogenic transcripts E6/E7 of HPV types 16, 18, 31, 33, 45, 58.

EP 1 997 914 A1

## Description

The present invention refers to the identification and quantification of oncogenic HPV nucleic acids by means of Real-Time PCR assays.

## Background of the Invention

In recent years it has been established that infection by oncogenic human papillomavirus (HPV) is a necessary condition for cervical carcinogenesis (Zur Hausen, 2002). The most frequent high risk HPV types are HPV 16, -18, -31, -33, -45 and -58. Persistent infection is considered to be the true precursor of neoplastic progression (Kjaer et al., 2002). Currently, however, HPV infections are monitored primarily by qualitative HPV DNA detection assays which are often not type specific and therefore in the clinical management of the patients do not distinguish between persistent and transient infections, the latter being extremely frequent in sexually active women. Qualitative unspecific viral detection therefore represents an inefficient means of identifying women at risk of developing cervical cancer (Ho et al, 1998; Jacobs et al, 2000). There is therefore a need to establish a suitable clinical marker able to distinguish persistent from transient infection in order to better identify those women at risk of neoplastic progression. Thus, HPV detection and typing techniques have been proposed as an adjunct to, or a replacement for, the current cytological screening regime. Clearly the success of such strategies will depend on the development of rapid, reliable, sensitive, and specific HPV detection methods applicable in the clinical setting. HPV viral load has been proposed as a surrogate marker of persistent infection (Ho et al, 1998) but its use in identifying women at risk of developing cervical carcinoma (CC) remains controversial (Josefsson et al, 2000; Lorincz et al, 2002; Dalstein et al, 2003; Gravitt et al, 2003; Moberg et al, 2004).

Few reliable quantitative PCR assays have been developed for the measurement of HPV load and this could account for the discrepancy in the results obtained in previous studies. The lack of standardisation of the methods used, particularly the chosen HPV sequence to be amplified and the way the number of cells per sample is determined, may be responsible for the controversial results (Moberg et al. 2004). There is great interest in using validated quantitative HPV assays in epidemiological studies to better define the evolution of HPV infection and lesion progression. Several real-time PCR assays have been developed to measure HPV-16 DNA load by adjusting the signal obtained for HPV-16 DNA with the amount of cellular DNA calculated from amplification of a human gene (Swan et al., 1997, 1999; Josefsson et al., 1999, 2000; Ylitalo et al., 2000; Peitsaro et al., 2002; Nagao et al., 2002; Beskow and Gyllensten, 2002). In addition to viral load, also the integration state of the HPV genome is known to have profound implications for patient prognosis. However, whether viral load or integration status of HPV is a risk factor for cervical cancer progression remains unclear due to conflicting results obtained using different methodologies in previous studies.

Furthermore, several studies have shown that in cervical carcinogenesis, the expression of HPV of specific E6 and E7 oncogene transcripts is required for cell transformation and immortalisation. Moreover, the presence of E6 and E7 has been found to increase with increasing severity of cervical disease (Kraus et al.,2004, Molden et al 2005). Consequently, persistent expression of these oncogenes may serve as an indicator of progression to neoplastic precursor lesions and invasive cancer (Sotlar et al., 1998), Detection of E6 and E7 mRNA may therefore be an additional valid tool in adjunct to viral DNA load in order to identify patients who are more likely to undergo disease progression. There is currently one commercially available HPV assay, PreTect® HPV-Proofer (NorChip), which detects E6/E7 mRNA transcripts for HPV 16, 18, 31,33 and 45 using NASBA method (Molden et al 2007). Initial data, on the prognostic value and specificity of this method for underlying disease is promising (Kraus et al.,2004, Cuschieri et al., 2004, Molden et al 2005) but the clinical value of this method compared with the quantification of HPV-DNA assays remains to be determined.

Finally a better understanding of the circulating HPV genotypes in different geographical areas has become very important, particularly in view of the recent introduction of bivalent HPV vaccines (types 16 and 18). Screening programmes will in fact need to be continued particularly in areas where other oncogenic genotypes are found to prevalent, as immunization will only protect against HPV types targeted by the vaccine (Roden 2006 review).

## Description of the invention

The present invention provides a system enabling to detect one or more high-risk HPV genotypes in clinical samples and to determine viral oncogenic activity by means of both specific DNA load and presence of E6/E7 mRNA. This system minimizes the number of parallel reactions performed for each sample and makes it suitable for use in routine screening of biological specimens such as cervical cytological samples, peripheral blood, urine, tissue biopsies and the like.

The invention more particularly provides a method for the identification and quantification of oncogenic HPV nucleic acids by means of Real-Time PCR assays comprising:

1) first line screening by means of a SYBR Green I Real-time PCR assay to determine the total viral load and to identify the presence of one or more of 13 high risk HPV genotypes in the sample;
2) second line assays to be applied to samples which have proved positive to first line screening, including:

- 4 independent TaqMan Real-time PCR assays to determine the presence and the viral load of the most common oncogenic HPV types: 16, 18 and/or 45, 31, 33 and/or 58.
- 6 independent SYBR Green I RT Real-time PCR assays to determine the presence in the sample of the oncogenic transcripts E6/E7 of HPV types 16, 18, 31, 33, 45, 58.

## Detailed description of the invention

The invention provides a method for the identification and quantification of oncogenic HPV nucleic acids comprising:

a) first line screening by means of a SYBR Green I Real-time PCR assay to determine the total viral DNA load and to identify the presence of one or more of 13 high risk HPV genotypes in clinical samples;
b) second line assays to be applied to samples which have proved positive to first line screening, including:

i) 4 independent TaqMan Real-time PCR assays to determine the presence and the viral load of the most common oncogenic HPV types: 16, 18 and/or 45, 31, 33 and/or 58;
ii) 6 independent SYBR Green I RT Real-time PCR assays to determine the presence in the sample of the oncogenic transcripts E6/E7 of HPV types 16, 18, 31, 33, 45, 58.

Real-Time PCR assays in virology are known and reviewed for instance by Mackay et al., 2002, Nucleic Acid Research, 30(6), 1292-1305.
Any clinical specimen obtainable from human tissues, organs or fluids may be used in the method of the invention.
Primers for the first SYBR Green I Real-Time PCR assay (step a) are reported below.

TABLE 1

| GENOTYPE | FORWARD | REVERSE |
|---|---|---|
| High-risk HPV | CGTCCAAAAGGAAACTGAGC (SEQ ID NO: 1) | GCACAGGGACATAACAATGG (SEQ ID NO: 2) |

The preferred primers for Real-time PCR (SYBR Green I method) for the determination of oncogenic transcripts E6/E7 (step b-ii) are reported below:

TABLE 2

| GENOTYPE | FORWARD | REVERSE |
|---|---|---|
| HPV 16 | CAGAGCTGCAAACAACTATA CATGATATA (SEQ ID NO: 3) | GTTAATACACCTCACGTCGCAGTA (SEQ ID NO: 4) |
| HPV 18 | TTACAGAGGTGCCTGCGGT (SEQ ID NO: 5) | TCTAAGTTTTTCTGCTGGATTCAA C (SEQ ID NO 6) |
| HPV 31 | CATTGGAAATACCCTACGAT GAA (SEQ ID NO: 7) | TTGACACGTTATACACCTTTGCAG (SEQ ID NO: 8) |
| HPV 33 | TTGAACTACAGTGCGTGGAA TG (SEQ ID NO: 9) | CAGCGCCCTCAGATCGTT (SEQ ID NO: 10) |
| HPV 45 | ACAAGACGTATCTATTGCCT GTGTATAT (SEQ ID NO: 11) | CCGCAGGCACCTCTGTG (SEQ ID NO: 12) |

(continued)

| GENOTYPE | FORWARD | REVERSE |
|---|---|---|
| HPV 58 | ATCGAATTGAAATGCGTTGAA (SEQ ID NO: 13) | GCACAGCGCCCTCAGAT (SEQ ID NO: 14) |

Finally, the preferred primers for the TaqMan Real-Time PCR assays (step b-i) are reported in table 3 of the following Experimental Section #1.

**Experimental Section #1**

**MATERIALS AND METHODS**

*Sample preparation from cervical samples*

Cervical cytological material was scraped from the endocervix using a rotary motion with a Cytobrusch (Digene Cervical sampler, Digene Corp., Gaithersburg, MD, USA) and then introduced in collection devices and rinsed into a vial containing 10 ml of phosphate-buffered saline (pH 7.4). Specimens were refrigerated and transported to the microbiology and virology laboratory within 1 hr on wet ice where they centrifuged at 1800 rpm for 10 min, and stored as split cellular pellets at -70˚C prior to nucleic acid extraction and HPV detection. Two different extraction methods (manual and automated) were compared in this study. Manual method includes organic extraction (phenol-chloroform) of the samples. Briefly, cellular pellets were resuspended in 450 μl of lysis solution containing 100 mM KC1, 10 mM Tris-HCl (pH 8.3), 2.5 mM $MgCl_2$, 0.5% (vol/vol) Tween 20, and 0.5% (vol/vol) Nonidet P-40. Samples were incubated at 95˚C for 30 min, mixed for 2 min, and digested with 50 μl of proteinase K (20 μg/ μl). After overnight incubation at 56˚C, samples were heated at 95˚C for 10 min to inactivate proteinase K. Phenol-chloroform extraction followed by high-salt isopropanol precipitation was performed as previously described (18), and purified material was resuspended in a final volume of 200 μl of AE buffer (5 mM Tris-HCl-0.5 mM EDTA). Automated method, a commercial kit for DNA extraction based on binding of nucleic acid to silica membrane (Macherey-Nagel Nucleospin robot 96 blood kit) was used. The 96-well plate format of the commercially available Macherey-Nagel Nucleospin kit allow integration into the workstation of a robotic liquid handler (Biomeck 2000, Beckman). To enable further automation of the procedure a set of 48 frozen cervical cytological samples previously tested for HPV-16 (24 samples HPV-16 positive and 24 HPV-16 negative), each divided into two pellets of equal aliquots, were extracted by the two methods.

Briefly, 200 μl of the resuspended cellular pellet, 25 μl of proteinase K, and 200 μl of Nucleospin lysis buffer BQ1 were added to each other and vortexed for 30 s.

To maximise DNA recovery from difficult samples (i.e. samples containing much emoglobin) the sample was then incubated for at least 1 hour at 56˚C. After incubation, 200 μl of 96 to 100% ethanol was added to each sample and the mixtures were vortexed again.

The Nucleospin plate was placed on top of the vacuum manifold, and the samples were distributed to the appropriate wells. A vacuum of 400 mbar was applied for 5 min or until the samples had been completely drawn through the filter. Each well was washed by adding 300 μl of Nucleospin B5 washing buffer. A vacuum of 400 mbar was then applied for 5 min, and the through-flow was discarded. Then 600 μl of Nucleospin buffer B5 was added to each well, and the vacuum was applied at 400 mb for 3 min. The vacuum was applied at 600 mbar for a further 10 min to remove any residual ethanol. The DNA was eluted by adding 200 μl of Nucleospin elution buffer BE directly to the silica membrane in each well. The plate was incubated with this solution at room temperature for 5 min, and then a vacuum of 400 mbar was applied for 10 min to elute the DNA.

*Quantification of HPV-DNA by Real-time quantitative PCR assays*

Plasmids containing HPV16, -18, -31, -33, -45, and -58 sequences were prepared by cloning from PCR products of clinical samples and a standard curve for each assay was set up. PCR products were cloned into the pCRII plasmid by using the TOPO-TA cloning kit (Invitrogen Corp., San Diego, Calif.) according to the manufacturer's instructions. The plasmids with integrated HPVs were purified with the Qiagen plasmid Maxiprep kit (Qiagen, Inc.), sequenced, and used to quantify the HPV-16, -18, -31, -33, -45, and -58 DNA.

Four independent real-time quantitative TaqMan PCR assays: HPV-16, 31, 18/45 and 33/58 (HPV-18, -45 and HPV-33, -58 were indistinguishable from each other). In order to normalize the HPV viral load to the number of cells in the sample, a quantitative detection system a single-copy human CCR5 gene was also used (Broccolo et al., 2005).

Cervical samples differ widely in the amount of DNA present. DNA from cervical samples was considered suitable for HPV viral load determination if the human CCR5 copy number for reaction was higher than $2x10^3$ (corresponding to $10^3$ cells for reaction). Amplification and detection were performed using using TaqMan technology and ABI Prism device (7900 SDS; Applied Biosystems, Forster City, CA). All reactions were optimized to obtain the best amplification kinetics under the same cycling conditions (2 min at 50˚C, 15 min at 95˚C, and 40 cycles of 15 s at 95˚C and 1 min per cycle at 60˚C) and composition of the reaction mixture. All reactions were performed in a final volume of 25 μl containing 100 mM (each) dATP, dCTP, and dGTP; 200 mM dUTP; 4 mM MgCl2; 1 X TaqMan buffer A (Applied Biosystems Foster City, Calif); 0.625 U of AmpliTaq Gold, 0.25 U of uracil-N-glycosylase; and 10 μl of DNA template. Tubes, containing all PCR components but without template DNA (denoted NTC reaction), were used to ensure that the reagents mix were free of contamination. Target-specific primers and probes were used at the final concentrations of 300 and 200 nM, respectively. The results of the experiment using these conditions are shown for HPV-16, HPV-33 and -58 in figure 1 and 2, respectively. The principle of the real-time PCR has been described elsewhere (Heid, et al. 1996). Briefly, the fluorescent signal (Rn) generated by the degradation of the hybridized probe is automatically calculated by a computer algorithm that normalizes the reporter emission signal first, by dividing it by the emission of a control dye (ROX) present in the PCR mix and then by subtracting all the background signals generated in the first 15 cycles of PCR. Then, the algorithm calculates the cycle of threshold (Ct) at which each PCR amplification reaches a threshold value (usually set at 10 times the standard deviation of the baseline signal) that is inversely proportional to the log number of target copies present in the sample. A standard curve was drawn using serial dilutions of known input target copies (x axis) versus the corresponding Ct values (y axis) using the least-squares fit method.

*Primer and probe design*

The target sequences for HPV-16, 18/45 and 33/58 are in the E1 open reading frame (ORF) while HPV-31 is in the E2 ORF. The primers and TaqMan probes (Table 3) were designed using Primer Express software (PE Biosystem, Foster City, Calif.).

As shown in table 3, two types of fluorogenic hybridization probes were utilized: two dual-labelled probes (in the assays to quantify HPV-18/45 and HPV-31) and two single-labelled probes (in the assays to quantify HPV-16 and HPV-33/58). The single-labelled probes have a minor groove binder (MGB) and non-fluorescent quencher at the 3'-end of the DNA sequence whereas dual-labelled probes have a fluorescent quencher (TAMRA) at the 3'-end of the DNA sequence. HVP-16 and -31 probes were labelled to the 5'-end of the DNA sequence with 5' fluorophore (FAM), while HPV-18/45 and 33/58 with 5' fluorophore (VIC). Finally, all sequences found in GenBank for each HPV genotype studied were aligned and primers and probe were chosen to avoid the mismatch between variants (for HPV-16 and HPV-31) and between genotypes (for HPV-18/45 and HPV-33/58). Moreover, both the E1 and E2 regions are highly conserved among different HPV variants. The alignment was performed to select a highly conserved region for each virus. Each primer and probe selected was checked against the alignment to ensure that each primer and probe was targeted to a conserved region in the respective alignments.

*HPV typing by $GP^+$-PCR system and INNO-LIPA analysis*

A total of 296 samples were tested for the presence of HPVs using two PCR primer set GP5+/GP6+ ($GP^+$-PCR); the conditions of the $GP^+$-PCR system were performed as described previously (Jacobs et al., 1995). The HPVs types detected using consensus primers were identified by sequencing of the PCR product using a fluorescently labelled dideoxy terminator kit (Amersham Pharmacia Biotech, Little Chalfont, United Kingdom). The sequences generated were compared to HPV sequences at GeneBank using the Fasta program (program manual for the Wisconsin package; Genetics Computer Group, Madison, Wis.). The limit of sensitivity for all genotypes studied was $10^4$ copies for reaction.

Another set of 31 samples were tested for the presence of HPVs using the INNO-LiPA HPV Genotyping v2 test (Innogenetics, Ghent, Belgium). following manufacturer's instructions. The INNO-LiPA HPV Genotyping v2 test enables specific detection of 24 HPV types, namely types 6, 11, 16, 18, 31, 33, 35, 39, 40, 42, 43, 44, 45, 51, 52, 53, 54, 56, 58, 59, 66, 68, 70, and 74, including multiple combinations. The test is based on PCR amplification of a 65 bp fragment within the L1 region of the HPV genome using the broad spectrum SPF10 biotinylated primers. Biotinylated amplicons are subsequently hybridized with HPV type-specific oligonucleotide probes which are immobilized as parallel lines on membrane strips. After hybridization and stringent washing, streptavidin-conjugated alkaline phosphatase is added and bound to any biotinylated hybrid formed. Incubation with BCIP/NBT chromogen yields a purple precipitate and the results can be visually interpreted (Kleter et al., 1999).

*Statistical analysis*

Accuracy, defined as the level of approximation of a measured value to a reference value taken as a "gold standard," was estimated by computing the arithmetic differences between DNA copy number evaluated by the real-time PCR assay and the theoretical number inferred from UV spectroscopy. The significance of systematic biases was assessed by a paired sample Student t test and was adjusted by covariance analysis, when needed. Repeatability and reproducibility were estimated by computing the coefficient of variation (CV) (the ratio between the standard deviation and the mean of repeated measurements) under different conditions. The significance of systematic biases was assessed by a paired sample Student *t* test and was adjusted by covariance analysis, when needed.

Normalization of HPV type-specific viral load was calculated as:

$$VL = \frac{Cn_{HPV}}{(Cn_{CCR5}/2)} \times 10^4 \text{ cells}$$

where VL is the number of HPV genomes per $10^4$ cells (corresponding to 2x $10^4$ CCR5 copies, $Cn_{HPV}$ is the number of HPV genomes and ($Cn_{CCR5}/2$) is the number of cells (corresponding to CCR5 copies number x 2).

**Results**

The present typing system was designed in order to allow quantification of the viral load for those high-risk HPV genotypes most frequently associated with cervical intraepithelial dysplasia and neoplasia. Although the prevalence of the oncogenic HPV types varies between studies, in the design of this typing system we focused on HPV-16, -18, -31, -33, 45 and 58. Four independent quantitative PCR fluorescent 5'-exonuclease reactions were designed and optimized using four separate aliquots of the same DNA specimen. The positions of the E1 and E2 primers and probes chosen for these assays are shown in Table 3.

**TABLE 3**. Primers and fluorescently labelled hybridization probes used for the quantification of DNA of HPV-16, -18, -31, -33, -45 and -58 by Real-time PCR.

| Target (Accession Number) | Sequence (5' to 3') | Labels (5', 3') Fluorophore |
|---|---|---|
| HPV-16 (NC 001526) | F16 (1126-1150) (SEQ ID NO 15): 5'-CGAAAGTATTTGGGTAGTCCACTTA-3'<br>R16 (1198-1228) (SEQ ID NO 16) 5'-CAGCTCTACTTTGTTTTTCTATACATATGG-3'<br>Probe[a] 16 (SEQ ID NO 17) 5'-AGTGAATGTGTAGACAATAA-3' | VIC, none |
| HPV-18 and -45 (AY262282) | F18/45 (2771-2793) (SEQ ID NO 18) 5'-TTTGAAAGGACATGGTCCAGAT 3'<br>R18/45 (2848-2831) (SEQ ID NO 19) 5'-CGTTCCGAAAGGGTTTCC 3'<br>Probe[b] 18/45 (SEQ ID NO 20) 5'-AGATTTGCACGAGGAAGAGGAAGATGC-3' | FAM, TAMRA |
| HPV-31 (J04353) | F31 (3355-3369) (SEQ ID NO 21) 5'-CCACCACATCGAATTCCAA -3'<br>R31 (3406-3390) (SEQ ID NO 22) 5'-CGCCGCACACCTTCAC-3'<br>Probe[b] 31 (SEQ ID NO 23) 5'-CCTGCGCCTTGGGCACC-3' | VIC, TAMRA |

(continued)

| Target (Accession Number) | Sequence (5' to 3') | Labels (5', 3') Fluorophore |
|---|---|---|
| HPV-33 and -58 (M12732) | F 33/58 (2701-2722) (SEQ ID NO 24) 5'-GGACGTGGTGCAAATTAGATTT-3'<br>R 33/58 (2767-2750) (SEQ ID NO 25) 5'-GTGCTGATATTTCCTCCATGGT-3'<br>Probe[a] 33/58 (SEQ ID NO 26) 5'-AGGAAGAGGACAAGGAA-3' | FAM, none |
| [a]MGB Probe: minor groove binder; [b]TAMRA probe | | |

*Strategies in probe and primers design*

To maximize sensitivity, DNA targets for real-time PCR amplifications were <100bp and the sequence of the probe was chosen so as to be close to the 3'forward primer (no further than 5 nt).

To reduce the number of assays, the primers and probes were chosen so as to quantify both HPV-18 and 45 in the same reaction tube as well as HPV-33 and 58. Shorter MGB probes were chosen for the detection of HPV-33and/or -58 as well as for HPV-16. This was found to be necessary in order to avoid the presence of mismatches between the sequences of HPV-33 and -58 as well as polymorphisms within sequences of different HPV-16 variants. By contrast, for HPV-18 and -45 it was not possible to avoid mismatches by the use of an MGB probe and therefore a longer dual-labelled probe was chosen in order to minimize the presence of mismatches (table 4).

**TABLE 4**. Localization of mismatch in different HPV genotypes

| Genotypes | Presence of mismatch | | |
|---|---|---|---|
| | Primer forward | probe | Primer reverse |
| HPV-18 vs. 45 | - | 2 | 1 |
| HPV-33 vs. 58 | 2 | - | 1 |

For HPV-31 a longer dual-labelled probe was also chosen as no mismatch were found in the probe among HPV-31 subtypes present in GenBank.

The presence and localization of mismatches in the two assays used to amplify HPV-18, -45 and -33, -58 are reported in table 4. Furthermore, special attention was taken in choosing sequences when there were found to be present one or more mismatches; in particular it was avoided to have in both probes and primers polymorphisms close within the same sequence and polymorphisms at the 5' end.

In addition, the kinetics profile of the two assays for the amplification of HPV-18 and -45 genotypes was compared; no significant differences were found between the plots generated for the two constructs (F = 1.30 [P = 0.23] and 1.35 [P = 0.22] for the intercept and the slope of the plots, respectively) (Fig. 1A); similar results were obtained for the amplification of HPV-33 and -58 genotypes (Fig. 1B).

*Dynamic range and analytical sensitivity and specificity*

In order to generate reference curves for the determination of HPV-16, -18, -31, -33, -45 and -58 viral loads (quantification), plasmids were quantified by UV spectroscopy. Thereafter, three distinct sets of 10-fold dilutions for each construct were prepared and amplified by PCR in the same run of the 7900 ABI Prism sequence detector system. A wide dynamic range characterized both assays, discriminating between $10^0$ and $10^6$ HPV genome equivalents/reaction. For all the systems generated, a strong linear relationship between the log of the starting copy number and the *Ct* values was obtained ($r^2$ = 0.99) (Fig. 2).

To better mimic the situation in which DNA is extracted from cells, we performed some experiments by adding human HPV-negative genomic DNA (100 ng of cervical cells DNA corresponding to 15,000 cellular genome equivalents) to a wide range of HPV-16 template DNA ($10^0$ to $10^6$ copy equivalent of DNA). The sensitivity and the performance of the assay were not affected by the presence of 100 ng of human genomic DNA (data not shown). Standard curves ranging from $10^1$ to $10^6$ copies per sample were constructed for each of the HPV types, or groups of HPV types, based on 12 independent measurements for each HPV copy number. Similar results were obtained for all others amplifications. By contrast, we observed that the sensitivity was affected when adding an excess of human HPV-negative genomic DNA (1 μg of cervical cells DNA corresponding to 150,000 cellular genome equivalents) to HPV-16 template DNA ($10^0$ to $10^2$ copy equivalent of DNA). To resolve this problem we increased by 5 sec in a stepwise fashion each annealing/ extension step for all cycles. However, when concentration of human genomic DNA was higher (>1 μg) the sample was diluted in order to have a final concentration not higher (equal or lower than) 1 μg/reaction (not higher that 1 μg/reaction). The specificity was tested by determining the ability of primer and probe combinations to discriminate plasmids with different HPV types. No aspecific signal was observed for the independent real-time PCR assays.

*Accuracy, repeatability, and reproducibility*

Next, we measured the accuracy of the TaqMan assays to quantify the HPV load for each genotype studied. For DNA inputs above 104 copies/reaction, the error observed (10 experiments performed with serial dilutions of the reference plasmids tested in triplicates) was negligible (2 to 9%), whereas it was greater (5 to 21%) for inputs below 104 copies/reaction. The repeatability and reproducibility of our *TaqMan* assays were assessed by calculating the CVs of the copy numbers determined experimentally experimentally (180 measurements for each reference DNA). Intraexperimental variability of assays was always below 20%, except for inputs of 10 genome equivalents/reaction (32 and 45%, respectively). Interexperimental variability was always below 40%, except for inputs of 10 genome equivalents/ reaction (49 and 74%, respectively).

*Real-time PCR assays in single tube*

In order to minimize the costs, we also tested a multiplex configuration in order to amplify multiple HPV targets in the same tube. All HPV types studied were analyzed in two reaction tubes (HPV-16 and -18/45, or 31 and - 33/58), allowing for quantification of three viral types in each reaction. Probes can be used separately or in combinations of up to two probes per assay. The assay is based on three parallel real-time PCRs from each patient sample: (i) reaction 1 detects and quantifies HPV-16, -18, and/or -45 (HPV18 and -45 were detected and quantified together) with two different fluorophores; (ii) reaction 2 detects and quantifies HPV-31, 33 and/or -58 (HPV33 and -58, were detected and quantified together), again with two different fluorophores; and (iii) reaction 3 detects and quantifies the amount of a human single-copy gene (CCR5 gene located on chromosome 3 codes for CC chemokine receptor 5; GenBank accession no. NC000003). Reaction 1 and 2, each includes a total of four PCR primers and two probes and reaction 3 includes two PCR primers and a single probe. Each assay was primarily set up as a monospecific assay which used a 10-fold dilution series of the plasmid containing the HPV fragment to be amplified. A standard curve was generated with the Ct values obtained, and this was used to optimize the PCR efficiency. The monospecific assay were then combined in two multiplex reactions and optimized.

An important aspect of a diagnostic assay is the ability to quantify individual HPV types in mixed infections. Thus we examined our real-time PCR assay's ability to correctly detect and quantify HPV viral loads in samples with multiple infections by producing synthetic mixtures of HPV-containing plasmids. Initially, the ability to correctly quantify the amount of HPV-16 in a background of HPV-18 was tested using the spike of $10^2$ copies of HPV-16: the assay was shown to be able to correctly estimate the amount of HPV-16 when the ratio of HPV-16 to HPV-18 was as low as 1:100 (Fig. 3) Similarly, in the reverse experiment (detection of HPV-18 in a background of HPV-16) the amount of HPV-18 was demonstrated to be accurately measured in a background of HPV-16, as long as the ratio of HPV-18 to HPV-16 was below 1:10 (Fig. 3). Similar results were obtained when HPV-31 was mixed with HPV-33. It was found that to correctly quantify HPV-31 with a background of HPV-33, the ratio of HPV-31 to HPV-33 must not be lower than 1:100. When we quantified HPV-33 in the presence of HPV-31, the ratio of HPV-33 to HPV-31 had to be lower than 1:10.

The specificity of the multiplex configuration was also tested by determining the ability of the primer and probe combinations in reaction 1 and reaction 2 to discriminate against plasmids with different HPV types. The specificity of the reagents in reaction 1 were tested by analyzing the signal with the HPV types detected in reaction 2 (with HPV-31, -33 and -58). Similarly, the specificity of the reagents in reaction 2 against the HPV types detected in reaction 1 (HPV-16, -18 and -45) was tested. No signal was observed with HPV-16, -18 and -45 at an initial concentration of $10^4$ viral copies (data not shown). The system was not tested for specificity with respect to other HPV types. However, the reaction 1 and reaction 2 were designed to be specific for the individual HPV types (using sequence alignments from a large number of HPV types) and reaction 3 for the nuclear gene (using GenBank and BLAST searches), respectively.

*Automated nucleic acid extraction by Biomeck 2000*

To enable further automation of the procedure a set of 48 frozen cervical cytological samples (each divided into two pellets of equal aliquots), were extracted by two different methods: manual extraction (phenol-chloroform protocol) and automated extraction (using an extraction kit integrated into a workstation of a robotic liquid handler). All steps (reagent mixing, incubation, washing and elution) excluding the loading of the sample and pellet lysis were performed by an automated station (Biomeck 2000 instrument).

Real-time PCR assays were carried out to compare the quality of DNA obtained by the Machery Nagel extraction method with that recovered by the classical phenol-chlorophorm extraction. The kinetics of the amplification plots showed no evidence of the presence of inhibitors in the DNA extracted by both methods. Twenty-four strongly positive for HPV-16 and 24 HPV negative samples were loaded on the Biomeck 2000 instrument.

After real-time PCR for HPV-16, none of the 24 negative samples gave a positive signal, while all 24 positive samples gave a nice amplification plot results indicating that no cross contamination had taken place during the automated procedure (data not shown).

*Real-time PCR assays compared to $GP^+$- PCR/sequencing system and INNO-LiPA HPV Genotyping*

46 samples were tested in parallel using $GP^+$- PCR and sequencing system and TaqMan assays. To avoid problems associated with sensitivity limit only samples that were TaqMan positive with $\geq 10^4$ copy/reaction were considered. Furthermore, DNA was extracted by the manual method and the real-time PCR assays were performed in a double-tube configuration. Overall, out of 46 samples positive by $GP^+$- PCR and sequencing system, 6 (13%) were found to be negative by Real-time PCR assays. The results are shown in table 5. Another 250 samples negative by $GP^+$- PCR and sequencing system were available, of these 15 (6%) resulted positive by the TaqMan assys with a viral load $\geq 10^4$ copies/reaction (up to sensitivity limit of the $GP^+$- PCR): 6 (2.4%) for HPV-16, 2 (1%) for HPV-18/45, 2 (1%) for HPV-31 and 5 (2%) for HPV-33/58.

Out of 31 samples positives by INNO-LiPA positive only 2 (6%), were found to be negative by real-time PCR assays (table 6).

All HPV-16 positive samples were confirmed by the TaqMan assay. The type most commonly missed was HPV-33/58 (5 cases).

**TABLE 5.** Comparison of results obtained by $GP^+$- PCR and sequencing system and Real-time PCR assay

| Analysis by: | HPV types identified | | | |
|---|---|---|---|---|
| | 16 | 31 | 18/45 | 33/58 |
| $GP^+$- PCR and sequencing[a] | 22 | 10 | 6 | 8 |
| TaqMan assay | 22 | 9 | 5 | 4 |
| No. of cases not confirmed by TaqMan assay (%) | 0 (0) | 1 (10) | 1[b] (17) | 4[c] (50) |

[a] For the comparison with the TaqMan assay was considered only the samples resulted with $\geq 10^4$ copy/reaction; [b]one of the five samples HPV-18 positive was not confirmed by TaqMan assay; [c] two of the four samples HPV-33 positive and two of the four samples HPV-58 positive were not confirmed by TaqMan assay.

**TABLE 6.** Comparison of results obtained by INNO-LiPA and Real-time PCR assay

| Analysis by: | HPV types identified | | | |
|---|---|---|---|---|
| | 16 | 31 | 18/45 | 33/58 |
| INNO-LiPA | 10 | 10 | 3 | 8 |
| TaqMan assay | 10 | 9 | 3 | 7 |
| No. of cases not confirmed by TaqMan assay (%) | 0 (0) | 1 (10) | 0 (0) | 1[a](13) |

[a] One of the seven samples HPV-58 positive was not confirmed by TaqMan assay

**Experimental Section #2**

MATERIAL AND METHODS

*Patients and clinical samples*

The study was performed on total of 593 patients. Of these 468 women were visited in the gynaecological outpatients clinic at San Gerardo Hospital, Monza and had pathologically proven cancerous or precancerous cervical lesions: 105 atypical squamous cells of undetermined significance (ASCUS), 200 low-grade (L-SIL), 150 high-grade (H-SIL) squamous intraepithelial lesions and 13 cervical cancer (CC). The age of these patients ranged between 19-76 years (median 37). 125 cervical samples, obtained from women aged between 20-65 (median 50), were found to have normal cytology. All specimens were collected between February 2005 and December 2006. Participating women gave informed consent and the study was approved by the local ethical committee.

*Cytological and histological examination of samples*

A Pap test was performed on all cohort participants during clinical investigation. Smears were classified into five ordered categories (negative, ASCUS, L-SIL, or H-SIL, CC). HPV-DNA testing was performed on all women. Colposcopy was carried out on all patients with a positive Pap smear and a punch biopsy was taken from suspect areas of the cervix. All histological assesments were performed by an experienced pathologist. The pathological diagnosis of cervical lesions was generally performed according to a five-grade framework: CIN I (grade I cervical intraepithelial neoplasia, characterized by condilomatous lesions and/or light dysplasia), CIN II (moderate dysplasia), CIN III (severe dysplasia), carcinoma in situ, and invasive carcinoma reflecting the Bethesda report model. If colposcopy findings were normal, no biopsy was taken and the case was classified as absence of CIN. When there were discrepancies between the histological and cytological findings, the worst result was regarded as the final diagnosis.

*Sample processing and DNA extraction*

Sample processing was performed as descripted in matherial and methods of the experimental section #1. DNA extraction was performed by a commercial kit for DNA extraction based on binding of nucleic acid to silica membrane (Macherey-Nagel Nucleospin robot 96 blood kit). The 96-well plate format of the commercially available Macherey-Nagel Nucleospin kit allow integration into the workstation of a robotic liquid handler (Biomeck 2000, Beckman) as reported in material and methods of the experimental section #1.

*Quantification of HPV-DNA by Real-time quantitative PCR assays*

High-risk HPV DNA loads were quantified by the use of four independent real-time quantitative TaqMan PCR assays: HPV-16, 31, 18/45 and 33/58. Details about these assays are described in the section #1. A CCR5 quantitative detection system was also used to quantify human genomic DNA in each sample and to normalize the viral load (Broccolo et al., 2005). The viral load is expressed as copy number for $10^4$ cells.

*Statistical analysis*

The Cochran-Armitage Trend test was used to detect an increasing trend in the proportion of HPV positive samples from women with normal cytology to those with CC.

$\chi^2$ test was used to analyze the significance of the different HPV genotypes prevalence in cervical samples from patients and controls. The two-tailed Student *t* test was used to evaluate the significance of differences in oncogenic HPV load between groups of samples. The total viral load, defined as the sum of the viral load for each type of virus where multiple viruses were found to be present, was calculated for each woman. An index of cograduation ($\gamma$ Goodman and Kruskall's index) was used to ordinal variables. This test was applied to measure the association (cograduation) grade between levels of DNA for each genotype and the severity of lesions. Goodman and Kruskall's index ($\gamma$) less than 0.3 represents fair too poor association, values of more than 0.3 represents good association. The level of statistical significance was set 0.05 and SPSS version 13 was used for all analyses.

**Results**

*Prevalence of the six oncogenic genotypes in pathological and normal cervical samples*

Frequency of type-specific HPV infection in Italy was investigated in all samples using Real-time PCR quantification assays. Positivity for one or more of the six oncogenic HPV genotypes evaluated was found in 288 (62%) and in 32 (26%) respectively of the pathological and normal cervical samples analysed. In women with cervical precancerous and cancerous lesions the overall prevalence of the HPV genotypes studied was of 25% for HPV-16, 13% for HPV-18/45, 23% for HPV-31 and 29% for HPV-33/58. As expected the prevalence of HPV types studied was markedly lower in normal samples with respect to each pathological category analyzed ($p < 0.05$) (table 7).

**TABLE 7.** Frequency of HPV-16, -18 and/or -45, -31, -33, and/or -58 in cervical samples from patients with diagnosis of ASCUS, LSIL, HSIL, cervical carcinoma and women with normal citology

| Diagnosis[a] | # of patients | # of cases of HPV-DNA positive for (%): | | | | # of patients positive for one or more genotypes (%) | P[d] |
|---|---|---|---|---|---|---|---|
| | | HPV-16 | HPV-18/45 | HPV-31 | HPV-33/58 | | |
| Normal | 125 | 9 (7) | 3 (2) | 17 (14) | 19 (15) | 32 (26) | 0.003 |
| ASCUS | 105 | 17 (16) | 12 (11) | 19 (18) | 35 (33) | 59 (56) | 0.006 |
| LSIL | 200 | 34 (17) | 24 (12) | 47 (24) | 57 (28) | 99 (50) | 0.006 |
| HSIL | 150 | 58 (39) | 23 (15) | 38 (25) | 39 (26) | 118 (79) | 0.006 |
| CC | 13 | 6 (46) | 1 (8) | 4 (31) | 3 (23) | 12 (92) | 0.006 |
| P value of Cochran-Armitage Trend test[c] | | <0.0001 | 0.0021 | 0.0060 | 0.1299 | <0.0001 | |

[a] ASCUS, atypical squamous cells of undetermined significance cells; L- or H-SIL, low- or high-grade squamous intraepithelial lesions; CC, cervical cancer; [b]The presence of one or more HPV genotypes was significantly higher in patients with ASCUS, LSIL, HSIL and CC as compared to women with normal cytology; [c]If statistically significant, the Cochran-Armitage Trend test detects an increasing trend in proportion of HPV positive from Normal to CC; [d]$\chi 2$ test was used to analyze the significance of the different HPV genotypes prevalence in cervical samples from patients and controls.

Positivity to each real-time PCR assay, alone and/or in combination with others, in patients with normal and pathological categories is shown in Figure 4.

*Type-specific HPV viral load associated with the different cytological/histological diagnosis*

The viral loads for oncogenic HPVs types studied are summarized in Table 8.

**TABLE 8**

| HPV type | n | 25% ILE | Mean | Median | 75% ILE | range |
|---|---|---|---|---|---|---|
| 16 | 124 | $4.4 \times 10^2$ | $4.3 \times 10^6$ | $2.1 \times 10^4$ | $1.9 \times 10^5$ | $1 - 3.5 \times 10^8$ |
| 18/45 | 63 | $1.3 \times 10^2$ | $5.8 \times 10^5$ | $9.3 \times 10^2$ | $1.05 \times 10^5$ | $10 - 1.9 \times 10^7$ |
| 31 | 125 | 14 | $4.1 \times 10^6$ | 80 | $2.4 \times 10^3$ | $3 - 1.9 \times 10^7$ |
| 33/58 | 153 | 50 | $2.3 \times 10^6$ | $2.9 \times 10^2$ | $4.2 \times 10^3$ | $11 - 2.9 \times 10^7$ |
| Two or more HPV types[a] | 115 | 80 | $6.9 \times 10^5$ | $2.2 \times 10^3$ | $1.08 \times 10^5$ | $1 - 3.5 \times 10^8$ |

No significant differences in the mean viral load values of the different HPV types considered were observed.

Overall, there was found to be a good association between HPVs total viral loads and severity of cytopathologic/histological findings (γ=0.46). As shown in figure 5, there was a linear increase in the median values of total viral loads for the HPV genotypes studied with disease progression.When viral load for each individual assay was analyzed there was found to be a significantly higher DNA load for HPV-16 and -31 genotypes in patients with CC when compared to women with normal cytology ($P < 0.05$); by contrast no statistically significant difference was found for HPV-18/45 and -33/58 (figure 5). A significant higher association between viral load and severity of disease was observed for HPV-31 and HPV-16 (γ=0.49 and 0.41, respectively ; a less significant association was found in the case of HPV-18/45 and HPV-33/58 (γ=0.19 and 0.02, respectively).

Finally, it was interesting to note that in 4 (12.5%) of the 32 HPV positive women with normal cytology, the viral DNA load was found to be higher than $10^4$ copies/$10^4$ cells.

Conclusion

This invention provides a system enabling in a step wise fashion to detect the presence of one or more high-risk HPV genotypes in clinical samples and to determine viral oncogenic activity by means of both specific DNA load and presence of E6/E7 mRNA.

This system provide a rapid and cost effective mean of carrying out routine screening of biological specimens for the presence of oncogenic HPV types.

Automated nucleic acid extraction, using a 96-well plate format integrated into a robotic liquid handler workstation, can further contribute to the rapidity of the screening and the possibility of a step wise performance of the assays based on their positivity can reduce the costs in the clinical management of patients.

**Description of the Figures**

**Figure 1**. Standard curve and Amplification plot for HPV-16 (A and B) and -31(C and D). Dilution series were made with plasmids containing $10^0$ to $10^6$ HPV-16 and HPV-31 copies. The threshold cycle ($C_t$) number is plotted against number of HPV-16 copies.

**Figure 2.** Comparison of the reference curves (A) for the HPV-18 and -45 and the amplification plot (B and C) for HPV-33 and -58 real-time PCR assays. The number of the plasmid molecules containing HPV-18 and -45 are indicated on the x axis, and the cycle of threshold (Ct) is indicated on the y axis; open triangles, for HPV18 (A) and HPV33 (B) TaqMan assays; open diamond, for HPV-45 (A) and HPV-58 (B) TaqMan assays. The equations for the HPV-18 and the HPV-45 constructs were y =38.83.- 3.32 log (x) and y =39.89 - 3.48 log (x), respectively. The equations for the HPV-13 and the HPV-58 constructs were y =38.83.- 3.32 log (x) and *y* =39.02 - 3.45 log (x), respectively.

**Figure 3**. Effect of cross talk due to emission spectrum overlap of the two dyes (FAM/VIC) on the outcome of in single tube (multiplex) assay. In the figures A, B and C are showed the detections of $10^2$ copies of HPV-16 (VIC dye) in a background different concentration of HPV-18 plasmids (FAM dye) while in the figures D, E and F are showed the detections of $10^2$ copies of HPV-18 (FAM dye) in a background different concentration of HPV-16 plasmids (VIC dye).

No effect of differential DNA concentration was showed when the differences between two target was lower 11og (A and D); effect of partial cross talk due to emission spectrum overlap was showed in B while effect of cross talk was showed in C; effect of cross talk was showed when the differences between target was higher of 2 log (E and F).

**Figure 4.** Prevalence of oncogenic HPV alone and in combination with others genotypes.

**Figure 5.** Distribution of HPV viral load, according to the degree of cervical lesion or cytological finding (normal, ASCUS, L-SIL, H-SIL and CC). Each box indicates the interquartile range. The lines that extend from each box indicate the extremes of values (whiskers represent the extreme values), and the line across each box indicates the median. The viral load was expressed in a log scale. A good association was showed between total oncogenic HPVs viral load and lesion degree (γ=0.46; figure A) as well as for HPV-16 (γ=0.41; B) and HPV-31 (γ=0.49; C); by contrast poor or none association was found for HPV18 or 45 (γ=0.19; D) and HPV-33 or 58 (γ=0.02; E).

**REFERENCES**

1. Andersson S et al. 2005 Br J Cancer: 92:2195-200.
2. Badaracco G et al. 2002. J Med Virol.: 67: 574-582.

3. Beskow AH et al. 2002. Int J Cancer: 101(6):526-31
4. Bosch FX et al. 2002. J Clin Pathol.: 55:244-65.
5. Bosch FX et al. 1995. J Natl Cancer Inst.: 87: 796-802.
6. Bosch FX et al. 2002. Virus Res.: 89: 183-190.
7. Boshart M et al. 1984. EMBO J.: 3: 1151- 1157.
8. Broccolo F et al. 2005. J Invest Dermatol. 124:1234-40.
9. Cullen AP et al. 1991. J Virol.: 65: 606-612.
10. Cuschieri KS et al. 2004. J Med Virol 2004;73:65-70.
11. Cuzick J et al. Br J Cancer: 69: 167-71.
12. Dalstein V et al. 2003. Int J Cancer: 106: 396-403.
13. Daniel B et al. J Gen Virol.: 78(Part 5): 1095-1101.
14. De Francesco MA et al. 2005. J Med Virol.: 75(4):588-92.
15. Flores R et al. 2006. Int J Cancer: 118: 1187-93.
16. Gravitt PE et al. J Virol Methods: 2003, 112(1-2):23-33.
17. Gravitt PE et al. 2003. Cancer Epidemiol Biomarkers Prev.:12: 477-484.
18. Gravitt PE et al. 2003. J Virol Methods: 112(1-2):23-33.
19. Hart KW et al. 2001. J Clin Microbiol.: 39(9):3204-12.
20. Heid CA et al. 1996. Genome Res.: 6:986-994.
21.Hesselink AT, et al. 2005. J Clin Microbiol. Sep;43(9):4868-71.
22. Ho GY,et al. 1998. N Engl J Med.: 338: 423-428.
23. Hudelist G,et al. 2004. Gynecol Oncol.: 92: 873-880.
24. Jacobs MV, et al. 1995. J Clin Microbiol 33:901-5.
25. Jacobs MV, et al. 2000. Int J Cancer 87: 221-227.
26. Josefsson AM, et al. 1999. J Clin Microbiol.: 37: 490-496.
27. Josefsson AM, et al. 2000. Lancet 355: 2189-2193.
28. Kjaer SK, et al. 2002. BMJ. 14;325(7364):572.
29. Kraus I, et al 2006. J Clin Microbiol. 44(4):1310-7.
30. Lorincz AT,et al. 2002. Lancet 360: 228-229.
31. Moberg M, et al. 2004. Int J Cancer: 112:854-9.
32. Moberg M et al. 2003. J Clin Microbiol. 41(7):3221-8.
33. Moberg M et al. 2004. Int J Cancer: 112: 854 -859.
34. Moberg M et al. 2005. Br J Cancer: 92: 891- 894.
35. Molden T et al. 2005. Cancer Epidemiol Biomarkers Prev 14 (2): 367-372.
36. Molden T et al. 2007. J Virol Methods; 142(1-2):204-12.
37. Nagao S et al. 2002. J Clin Microbiol. 40(3):863-7.
38. Peitsaro P et al. 2002. J Clin Microbiol.: 40: 886-891.
39. Pirami L et al. 1997. J Clin Pathol.: 50: 600-604.
40. Rasu M et al. 2005. Scand J Infect Dis;37:476-481.
41. Roden R, Wu TC. 2006. Nature Reviews Cancer;6:753-763.
42. Schiffman MH et al. 1993. J Natl Cancer Inst.: 85: 958-964.
43. Schlecht NF et al. 2003. J Natl Cancer Inst.: 95: 1336-1343.
44. Sherman ME et al. 2002. J Natl Cancer Inst.: 94: 102-107.
45. Sherman ME et al. 2003. Cancer Epidemiol Biomarkers Prev.: 12: 1038-1044.
46. Sun CA et al. 2002. Int J Gynaecol Obstet.: 76: 41-47.
47. Swan DC et al. 1997. J Clin Microbiol: 35: 886-91.
48. Swan DC et al. 1999. J Clin Microbiol: 37: 1030-4.
49. van Duin M et al. 2002. Int J Cancer 98: 590- 595.
50. Walboomers JM et al. 1999. J Pathol: 189: 12-19.
51. Wang-Johanning F et al. 2002. Cancer: 94:2199-210.
52. Ylitalo N et al. 2000. Cancer Res 60: 6027-6032.
53. Woodman CBJ et al. 2007. Nature Reviews Cancer: 7:11-22.
54. Zerbini M et al. 2001. J Clin Pathol: 54:377-380.
55. Zur Hausen H. 2002.Nat Rev Cancer:2:342-50.

## Claims

1. A method for the identification and quantification of oncogenic HPV nucleic acids comprising:

   a) first line screening by means of a SYBR Green I Real-time PCR assay to determine the total viral DNA load and to identify the presence of one or more of 13 high risk HPV genotypes in clinical samples;
   b) second line assays to be applied to samples positives for a):

   - 4 independent TaqMan Real-time PCR assays to determine the presence and the viral load of the most common oncogenic HPV types: 16, 18 and/or 45, 31, 33 and/or 58;
   - 6 independent SYBR Green I RT Real-time PCR assays to determine the presence in the sample of the oncogenic transcripts E6/E7 of HPV types 16, 18, 31, 33, 45, 58.

2. A method according to claim 1 wherein said sample can be any biological specimens such as cervical cytological samples, peripheral blood, urine, tissue biopsies and the like.

3. A method according to claim 1 or 2, wherein the primers used in step a) have the sequences SEQ ID NO: 1 and SEQ ID NO: 2.

4. A method according to any one of claims 1-3 wherein the primers used in the TaqMan Real-Time PCR assay have the sequences SEQ ID 15-SEQ ID 26.

5. A method according to any one of claims 1-4, wherein the primers used in the SYBR Green Real-time PCR assay in step b have the sequences SEQ ID 3-SEQ ID 14.

6. A kit for performing the methods of claims 1-5 including the primers of claims 3-5 and suitable buffers, probes and labels.

**Figure 1**

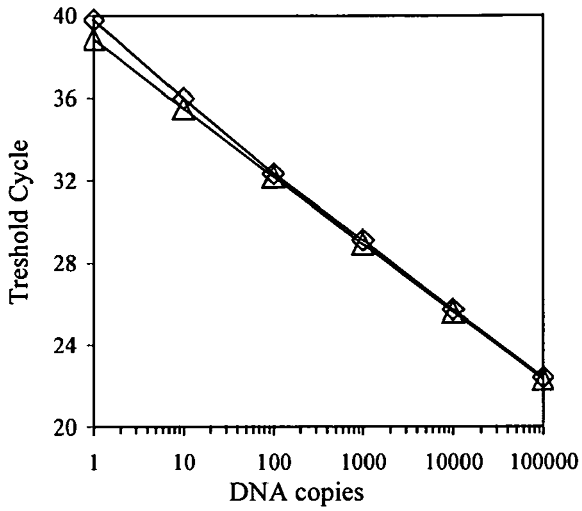
40
36
32
28
24
20
Treshold Cycle
1
10
100
1000
10000
100000
DNA copies

A

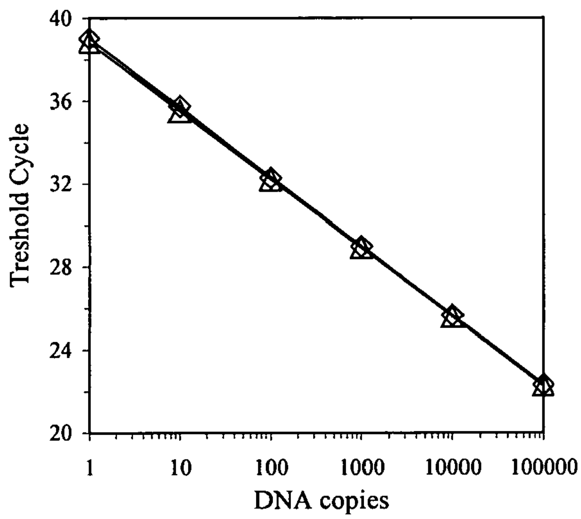
40
36
32
28
24
20
Treshold Cycle
1
10
100
1000
10000
100000
DNA copies

B

**Figure 2**

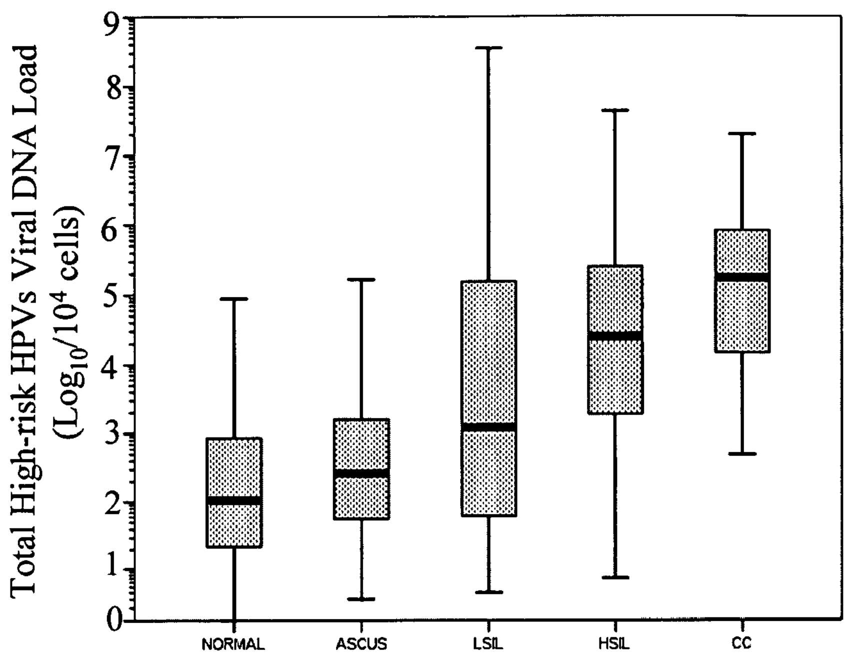
Total High-risk HPVs Viral DNA Load
(Log10/10^4 cells)
9
8
7
6
5
4
3
2
1
0
NORMAL
ASCUS
LSIL
HSIL
CC

**Figure 3**

HPV16 detection (VIC dye labels)

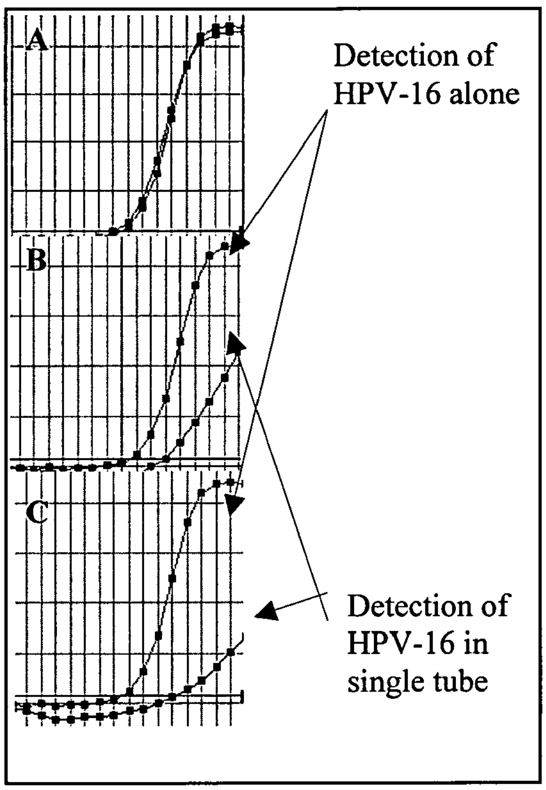


HPV-18 detection (FAM dye labels)

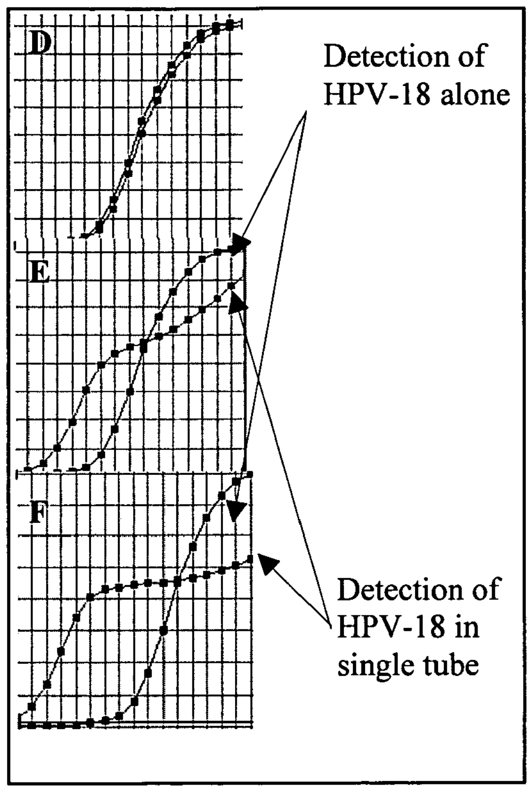

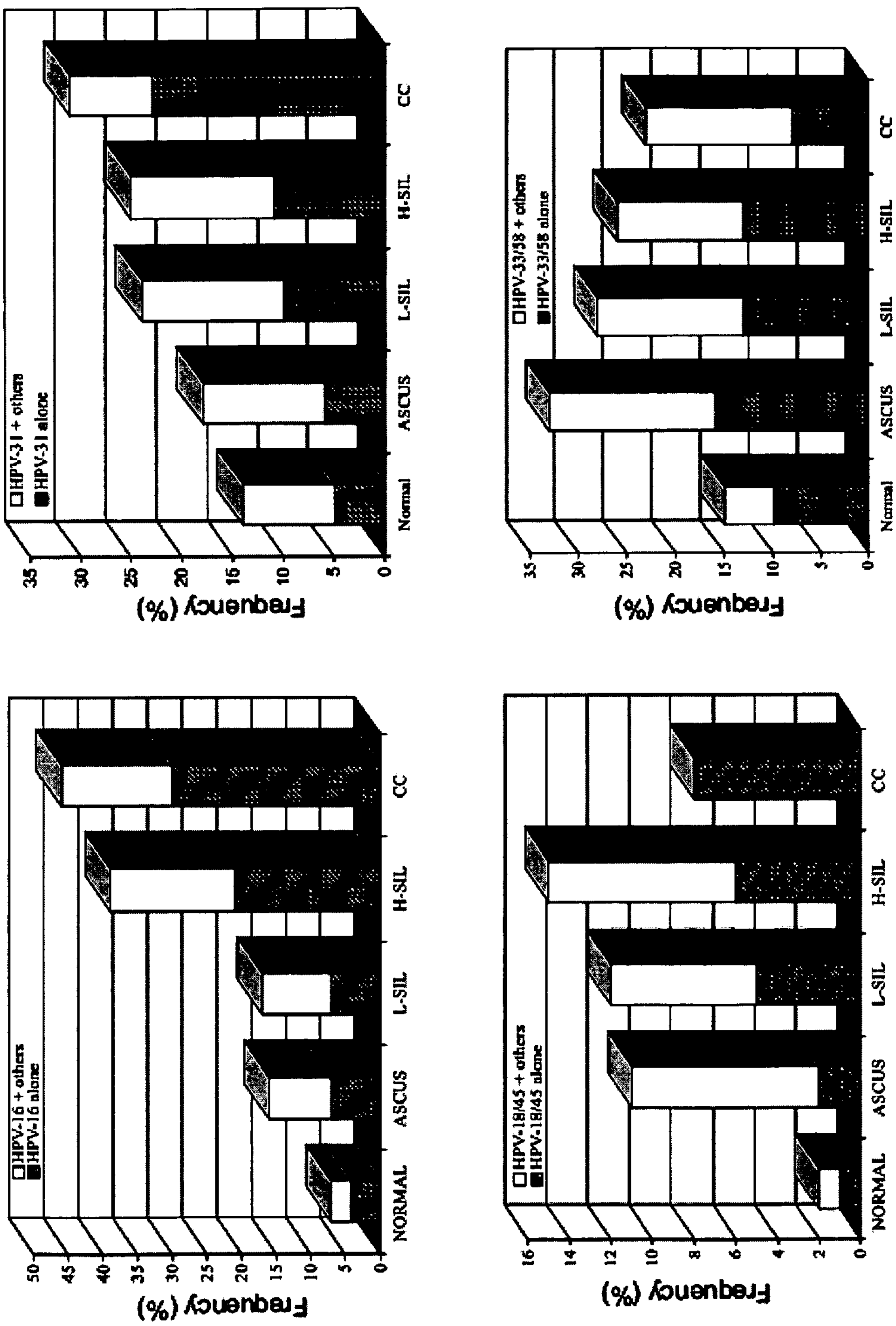
HPV-16 + others
HPV-16 alone
HPV-31 + others
HPV-31 alone
HPV-18/45 + others
HPV-18/45 alone
HPV-33/58 + others
HPV-33/58 alone
Frequency (%)
NORMAL
ASCUS
L-SIL
H-SIL
CC

Figure 4

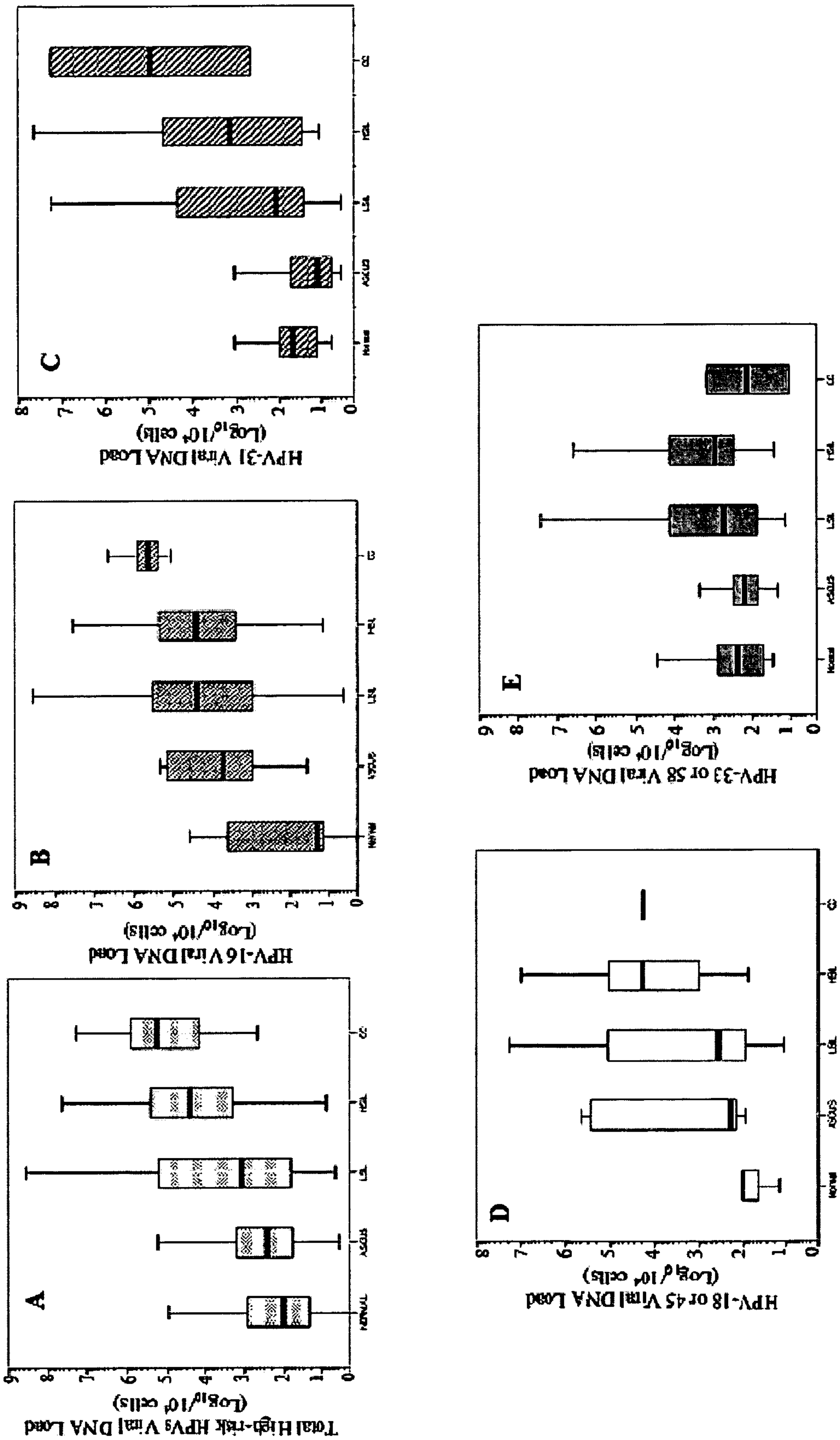
A
Total High-risk HPVs Viral DNA Load
(Log10/10⁴ cells)
B
HPV-16 Viral DNA Load
(Log10/10⁴ cells)
C
HPV-31 Viral DNA Load
(Log10/10⁴ cells)
D
HPV-18 or 45 Viral DNA Load
(Log10/10⁴ cells)
E
HPV-33 or 58 Viral DNA Load
(Log10/10⁴ cells)

Figure 5

European Patent Office

# EUROPEAN SEARCH REPORT

Application Number
EP 07 10 9454

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/031416 A (QUANTOVIR AB [SE]; GYLLENSTEN ULF [SE]; MOBERG MARTIN [SE]) 15 April 2004 (2004-04-15)<br>* the whole document *<br>----- | 1-6 | INV.<br>C12Q1/70 |
| X | PEITSARO PANU ET AL: "Integrated human papillomavirus type 16 is frequently found in cervical cancer precursors as demonstrated by a novel quantitative real-time PCR technique."<br>JOURNAL OF CLINICAL MICROBIOLOGY MAR 2002, vol. 40, no. 3, March 2002 (2002-03), pages 886-891, XP002456424<br>ISSN: 0095-1137<br>* the whole document *<br>----- | 1-6 | |
| Y | SUN ZHEN ET AL: "Multiplex locked nucleic acid probes for analysis of hepatitis B virus mutants using real-time PCR"<br>GENOMICS,<br>vol. 89, no. 1, January 2007 (2007-01), pages 151-159, XP002456516<br>ISSN: 0888-7543<br>* the whole document *<br>----- | 1-6 | TECHNICAL FIELDS SEARCHED (IPC)<br>C12Q |
| Y | US 2005/175987 A1 (JANSEN KATHRIN [US] ET AL) 11 August 2005 (2005-08-11)<br>* the whole document *<br>----- | 1-6 | |
| D,Y | BROCCOLO FRANCESCO ET AL: "Additional evidence that pityriasis rosea is associated with reactivation of human herpesvirus-6 and-7"<br>JOURNAL OF INVESTIGATIVE DERMATOLOGY, vol. 124, no. 6, June 2005 (2005-06), pages 1234-1240, XP002456425<br>ISSN: 0022-202X<br>* the whole document *<br>-----<br>-/-- | 1-6 | |

The present search report has been drawn up for all claims

| Place of search | Date of completion of the search | Examiner |
|---|---|---|
| The Hague | 25 October 2007 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

European Patent Office

# EUROPEAN SEARCH REPORT

Application Number
EP 07 10 9454

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | TUCKER RUTH ANN ET AL: "Real-time PCR-based fluorescent assay for quantitation of human papillomavirus types 6, 11, 16 and 18" MOLECULAR DIAGNOSIS, NAPERVILLE, IL, US, vol. 6, no. 1, 1 March 2001 (2001-03-01), pages 39-47, XP002970543 ISSN: 1084-8592 * the whole document * ----- | 1-6 | |
| Y | JOSEFSSON AGNETHA ET AL: "Detection and quantitation of human papillomavirus by using the fluorescent 5' exonuclease assay" JOURNAL OF CLINICAL MICROBIOLOGY, WASHINGTON, DC, US, vol. 37, no. 3, March 1999 (1999-03), pages 490-496, XP002193785 ISSN: 0095-1137 * the whole document * ----- | 1-6 | TECHNICAL FIELDS SEARCHED (IPC) |
| Y | WO 2006/084155 A (PATTERSON BRUCE K [US]) 10 August 2006 (2006-08-10) * the whole document * ----- | 1-6 | |
| A | SWAN DAVID C ET AL: "A sensitive, type-specific, fluorogenic probe assay for detection of human papillomavirus DNA" JOURNAL OF CLINICAL MICROBIOLOGY, WASHINGTON, DC, US, vol. 35, no. 4, April 1997 (1997-04), pages 886-891, XP002970544 ISSN: 0095-1137 * the whole document * ----- | 1-6 | |

The present search report has been drawn up for all claims

2

| Place of search | Date of completion of the search | Examiner |
|---|---|---|
| The Hague | 25 October 2007 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 10 9454

This annex lists the patent family members relating to the patent documents cited in the above-mentioned European search report.
The members are as contained in the European Patent Office EDP file on
The European Patent Office is in no way liable for these particulars which are merely given for the purpose of information.

25-10-2007

| Patent document cited in search report | Publication date | Patent family member(s) | Publication date |
|---|---|---|---|
| WO 2004031416 A | 15-04-2004 | AU 2003267898 A1 | 23-04-2004 |
| | | CA 2501030 A1 | 15-04-2004 |
| | | EP 1546413 A1 | 29-06-2005 |
| | | JP 2006500952 T | 12-01-2006 |
| | | US 2007037137 A1 | 15-02-2007 |
| US 2005175987 A1 | 11-08-2005 | NONE | |
| WO 2006084155 A | 10-08-2006 | AU 2006210483 A1 | 10-08-2006 |
| | | US 2006172285 A1 | 03-08-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MACKAY et al.** *Nucleic Acid Research,* 2002, vol. 30 (6), 1292-1305
- **ANDERSSON S et al.** *Br J Cancer,* 2005, vol. 92, 2195-200
- **BADARACCO G et al.** *J Med Virol.,* 2002, vol. 67, 574-582
- **BESKOW AH et al.** *Int J Cancer,* 2002, vol. 101 (6), 526-31
- **BOSCH FX et al.** *J Clin Pathol.,* 2002, vol. 55, 244-65
- **BOSCH FX et al.** *J Natl Cancer Inst.,* 1995, vol. 87, 796-802
- **BOSCH FX et al.** *Virus Res.,* 2002, vol. 89, 183-190
- **BOSHART M et al.** *EMBO J.,* 1984, vol. 3, 1151-1157
- **BROCCOLO F et al.** *J Invest Dermatol.,* 2005, vol. 124, 1234-40
- **CULLEN AP et al.** *J Virol.,* 1991, vol. 65, 606-612
- **CUSCHIERI KS et al.** *J Med Virol,* 2004, vol. 73, 65-70
- **CUZICK J et al.** *Br J Cancer,* vol. 69, 167-71
- **DALSTEIN V et al.** *Int J Cancer,* 2003, vol. 106, 396-403
- **DANIEL B et al.** *J Gen Virol.,* vol. 78 (5), 1095-1101
- **DE FRANCESCO MA et al.** *J Med Virol.,* 2005, vol. 75 (4), 588-92
- **FLORES R et al.** *Int J Cancer,* 2006, vol. 118, 1187-93
- **GRAVITT PE et al.** *J Virol Methods,* 2003, vol. 112 (1-2), 23-33
- **GRAVITT PE et al.** *Cancer Epidemiol Biomarkers Prev.,* 2003, vol. 12, 477-484
- **HART KW et al.** *J Clin Microbiol.,* 2001, vol. 39 (9), 3204-12
- **HEID CA et al.** *Genome Res.,* 1996, vol. 6, 986-994
- **HESSELINK AT et al.** *J Clin Microbiol.,* September 2005, vol. 43 (9), 4868-71
- **HO GY.** *N Engl J Med.,* 1998, vol. 338, 423-428
- **HUDELIST G.** *Gynecol Oncol.,* 2004, vol. 92, 873-880
- **JACOBS MV et al.** *J Clin Microbiol,* 1995, vol. 33, 901-5
- **JACOBS MV et al.** *Int J Cancer,* 2000, vol. 87, 221-227
- **JOSEFSSON AM et al.** *J Clin Microbiol.,* 1999, vol. 37, 490-496
- **JOSEFSSON AM et al.** *Lancet,* 2000, vol. 355, 2189-2193
- **KJAER SK et al.** *BMJ,* vol. 325 (7364), 572
- **KRAUS I et al.** *J Clin Microbiol.,* 2006, vol. 44 (4), 1310-7
- **LORINCZ AT.** *Lancet,* 2002, vol. 360, 228-229
- **MOBERG M et al.** *Int J Cancer,* 2004, vol. 112, 854-9
- **MOBERG M et al.** *J Clin Microbiol.,* 2003, vol. 41 (7), 3221-8
- **MOBERG M et al.** *Int J Cancer,* 2004, vol. 112, 854-859
- **MOBERG M et al.** *Br J Cancer,* 2005, vol. 92, 891-894
- **MOLDEN T et al.** *Cancer Epidemiol Biomarkers Prev,* 2005, vol. 14 (2), 367-372
- **MOLDEN T et al.** *J Virol Methods,* 2007, vol. 142 (1-2), 204-12
- **NAGAO S et al.** *J Clin Microbiol.,* 2002, vol. 40 (3), 863-7
- **PEITSARO P et al.** *J Clin Microbiol.,* 2002, vol. 40, 886-891
- **PIRAMI L et al.** *J Clin Pathol.,* 1997, vol. 50, 600-604
- **RASU M et al.** *Scand J Infect Dis,* 2005, vol. 37, 476-481
- **RODEN R ; WU TC.** *Nature Reviews Cancer,* 2006, vol. 6, 753-763
- **SCHIFFMAN MH et al.** *J Natl Cancer Inst.,* 1993, vol. 85, 958-964
- **SCHLECHT NF et al.** *J Natl Cancer Inst.,* 2003, vol. 95, 1336-1343
- **SHERMAN ME et al.** *J Natl Cancer Inst.,* 2002, vol. 94, 102-107
- **SHERMAN ME et al.** *Cancer Epidemiol Biomarkers Prev.,* 2003, vol. 12, 1038-1044
- **SUN CA et al.** *Int J Gynaecol Obstet.,* 2002, vol. 76, 41-47
- **SWAN DC et al.** *J Clin Microbiol,* 1997, vol. 35, 886-91
- **SWAN DC et al.** *J Clin Microbiol,* 1999, vol. 37, 1030-4
- **VAN DUIN M et al.** *Int J Cancer,* 2002, vol. 98, 590-595
- **WALBOOMERS JM et al.** *J Pathol,* 1999, vol. 189, 12-19

- **WANG-JOHANNING F et al.** *Cancer,* 2002, vol. 94, 2199-210
- **YLITALO N et al.** *Cancer Res,* 2000, vol. 60, 6027-6032
- **WOODMAN CBJ et al.** *Nature Reviews Cancer,* 2007, vol. 7, 11-22
- **ZERBINI M et al.** *J Clin Pathol,* 2001, vol. 54, 377-380
- **ZUR HAUSEN H.** *Nat Rev Cancer,* 2002, vol. 2, 342-50

SEQUENCE LISTING

<110> Università degli Studi di Milano-Bicocca

<120> Identification and quantification of oncogenic HPV nucleic acids by means of Real-Time PCR assays.

<130> UNIMIB

<160> 26

<170> PatentIn version 3.3

<210> 1
<211> 20
<212> DNA
<213> Synthetic oligonucleotide

<400> 1
cgtccaaaag gaaactgagc 20

<210> 2
<211> 20
<212> DNA
<213> synthetic oligonucleotide

<400> 2
gcacagggac ataacaatgg 20

<210> 3
<211> 29
<212> DNA
<213> synthetic oligonucleotide

<400> 3
cagagctgca aacaactata catgatata 29

<210> 4
<211> 24
<212> DNA
<213> synthetic oligonucleotide

<400> 4
gttaatacac ctcacgtcgc agta 24

<210> 5
<211> 19
<212> DNA
<213> synthetic oligonucleotide

<400> 5
ttacagaggt gcctgcggt 19

<210> 6
<211> 25
<212> DNA
<213> synthetic oligonucleotide

<400> 6
tctaagtttt tctgctggat tcaac 25

<210> 7
<211> 23
<212> DNA
<213> synthetic oligonucleotide

<400> 7
cattggaaat accctacgat gaa 23

<210> 8
<211> 24
<212> DNA
<213> synthetic oligonucleotide

<400> 8
ttgacacgtt atacaccttt gcag 24

<210> 9
<211> 22
<212> DNA
<213> synthetic oligonucleotide

<400> 9
ttgaactaca gtgcgtggaa tg 22

<210> 10
<211> 18
<212> DNA
<213> synthetic oligonucleotide

<400> 10
cagcgccctc agatcgtt 18

<210> 11
<211> 28
<212> DNA
<213> synthetic oligonucleotide

<400> 11
acaagacgta tctattgcct gtgtatat 28

<210> 12
<211> 17
<212> DNA
<213> synthetic oligonucleotide

<400> 12
ccgcaggcac ctctgtg 17

<210> 13
<211> 21
<212> DNA
<213> synthetic oligonucleotide

<400> 13
atcgaattga aatgcgttga a 21

<210> 14
<211> 17
<212> DNA
<213> synthetic oligonucleotide

<400> 14
gcacagcgcc ctcagat 17

<210> 15
<211> 25
<212> DNA
<213> synthetic oligonucleotide

<400> 15
cgaaagtatt tgggtagtcc actta 25

<210> 16
<211> 30
<212> DNA
<213> synthetic oligonucleotide

<400> 16
cagctctact ttgtttttct atacatatgg 30

<210> 17
<211> 20
<212> DNA
<213> synthetic oligonucleotide

<400> 17
agtgaatgtg tagacaataa 20

<210> 18
<211> 22
<212> DNA
<213> synthetic oligonucleotide

<400> 18
tttgaaagga catggtccag at 22

<210> 19
<211> 18
<212> DNA
<213> synthetic oligonucleotide

<400> 19
cgttccgaaa gggtttcc 18

<210> 20
<211> 27
<212> DNA
<213> synthetic oligonucleotide

<400> 20
agatttgcac gaggaagagg aagatgc 27

<210> 21
<211> 19
<212> DNA
<213> synthetic oligonucleotide

<400> 21
ccaccacatc gaattccaa 19

<210> 22
<211> 16
<212> DNA
<213> synthetic oligonucleotide

<400> 22
cgccgcacac cttcac 16

<210> 23
<211> 17
<212> DNA
<213> synthetic oligonucleotide

<400> 23
cctgcgcctt gggcacc 17

<210> 24
<211> 22
<212> DNA
<213> synthetic oligonucleotide

<400> 24
ggacgtggtg caaattagat tt 22

<210> 25
<211> 22
<212> DNA
<213> synthetic oligonucleotide

<400> 25
gtgctgatat ttcctccatg gt 22

<210> 26
<211> 17
<212> DNA
<213> synthetic oligonucleotide

<400> 26
aggaagagga caaggaa 17